Europäisches Patentamt

European Patent Office

Office européen des brevets

(19)

(11) Publication number: **0 107 972**

**A1**

(12) **EUROPEAN PATENT APPLICATION**

(21) Application number: **83306534.5**

(22) Date of filing: **27.10.83**

(51) Int. Cl.³: **C 07 C 57/58**
**C 07 C 51/487, C 07 B 19/00**

(30) Priority: **29.10.82 JP 191142/82**

(43) Date of publication of application:
**09.05.84 Bulletin 84/19**

(84) Designated Contracting States:
**DE FR GB IT NL**

(71) Applicant: **SUMITOMO CHEMICAL COMPANY, LIMITED**
**15 Kitahama 5-chome Higashi-ku**
**Osaka-shi Osaka-fu(JP)**

(72) Inventor: **Takuma, Kenzi**
**6-3, Nishimami-2-chome Kashibacho**
**Kitakatsuragi-Gun Nara-ken(JP)**

(72) Inventor: **Morino, Haruki**
**12-12, Yanagisawa-5-chome**
**Hoya-shi(JP)**

(74) Representative: **Paget, Hugh Charles Edward et al,**
**MEWBURN ELLIS & CO. 2/3 Cursitor Street**
**London EC4A 1BQ(GB)**

(54) A method for optical resolution of alpha-isopropyl-p-chlorophenylacetic acid.

(57) A method for optical resolution of α-isopropyl-p-chlorophenylacetic acid comprises reacting the α-isopropyl-p-chlorophenylacetic acid with (+)- or (−)-α-phenyl-β-(p-tolyl)ethylamine of not less than 95% in optical purity to selectively crystallize the (+)-α-phenyl-β-(p-tolyl)ethylamine salt of (+)-α-isopropyl-p-chlorophenylacetic acid or the (−)-α-phenyl-β-(p-tolyl)ethylamine salt of (−)-α-isopropyl-p-chlorophenylacetic acid, and then collecting and decomposing the resulting salt to obtain (+)- or (−)-α-isopropyl-p-chlorophenylacetic acid.

The α-isopropyl-p-chlorophenylacetic acid is useful as a carboxylic acid moiety of, for example, pyrethroid type insecticidal esters such as fenvalerate.

EP 0 107 972 A1

# A METHOD FOR OPTICAL RESOLUTION OF

## α-ISOPROPYL-p-CHLOROPHENYLACETIC ACID

The present invention relates to a method for optical resolution of α-isopropyl-p-chlorophenylacetic acid (hereinafter referred to as ICPA). More particularly, it relates to a method for the optical resolution of ICPA by using an optically active α-phenyl-β-(p-tolyl)ethylamine (hereinafter referred to as PTE) as an optical resolving agent.

ICPA, an object of the present invention, is the carboxylic acid moiety of, for example, pyrethroid type insecticidal esters such as fenvalerate. The followings are well known on said ICPA: Its (-)-isomer has little insecticidal activity as the ester, while the ester of its (+)-isomer has an insecticidal activity of about two times that of the (±)-isomer [Japanese Patent Application Kokai (Laid-open) No. 136245/1980]. There is a strong demand, therefore, for the development of a method to obtain said (+)-isomer of ICPA more advantageously in industry.

It is hitherto known that an optically active PTE or α-phenethylamine is used as an optical resolving agent for ICPA [Japanese Patent Application Kokai (Laid-open) No. 25544/1975]. In order to obtain pure (+)-ICPA of high optical purity, however, it is necessary to use large quantities of solvent and to repeat recrystallization several times; and when (+)-PTE of low optical purity is used, the filterability of the salt crystal becomes extremely poor, thereby making it difficult to obtain (+)-ICPA of high optical purity. It may therefore be said that, in order to obtain (+)-ICPA of high optical purity, this method is still not always satisfactory in that it is obliged to use (+)-PTE of high optical purity. Also, as an improved method to solve the foregoing problems, it is known that, by using a mixed solvent comprising hydrophobic and hydrophilic organic solvents and/or water as a solvent for resolution, the optical purity of (+)-ICPA obtained improves, the amount of solvent used can be decreased, and besides that, if (+)-PTE used is of low optical purity, there is not a lowering in the filterability of the salt crystal [Japanese Patent Application Kokai (Laid-open) No. 136245/1980].

In the course of a further study on a method for the optical resolution of ICPA, the present inventors found that, in the combination of ICPA and PTE, a complex salt is formed at a 1 : 1 : 1 : 1 molar ratio of (+)-ICPA : (-)-ICPA : (+)-PTE : (-)-PTE, said salt is easy to deposit as crystal because of its low solubility, and that the presence of said salt makes the filterability of the salt crystal markedly poor in the operation of optical resolution.

This means that: In the resolution of (±)-ICPA, the use of (+)-PTE of low optical purity produces the above complex salt corresponding to the amount of coexisting (-)-PTE, and because of said salt being lower in solubility than the (+)-ICPA-(+)-PTE salt and easy to deposit as crystal, it results that when the crystal portion is collected by filtration and decomposed, ICPA of lowered optical purity is obtained; and for the same reason, it is also difficult to obtain ICPA in high purity and high yield by recrystallization.

As a result of an extensive study based on this novel fact, the present inventors found that, by carrying out the optical resolution of ICPA with (+)-PTE of 95% or more in optical purity, (+)-ICPA of high optical purity can be obtained in high yield without special purification and also with a good filterability of the crystal. The present inventors thus attained to the present invention.

Next, the method of the present invention will be illustrated in detail. ICPA and PTE each has one asymmetric carbon atom in the molecule, and there are four optical isomers for the salt. Hereinafter, four said optical isomers are abbreviated as follows:

| (+)-ICPA-(+)-PTE salt | (+)(+) |
|---|---|
| (-)-ICPA-(+)-PTE salt | (-)(+) |
| (+)-ICPA-(-)-PTE salt | (+)(-) |
| (-)-ICPA-(-)-PTE salt | (-)(-) |

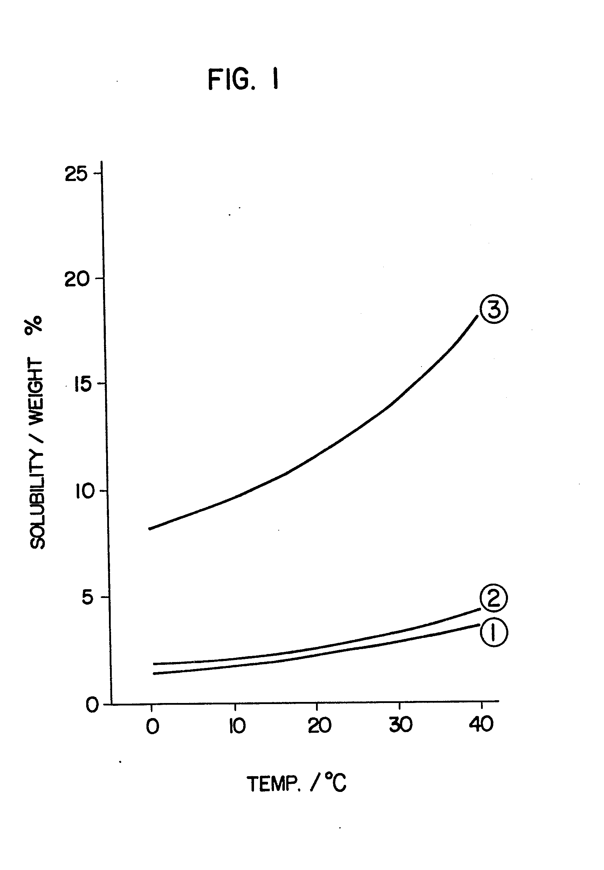

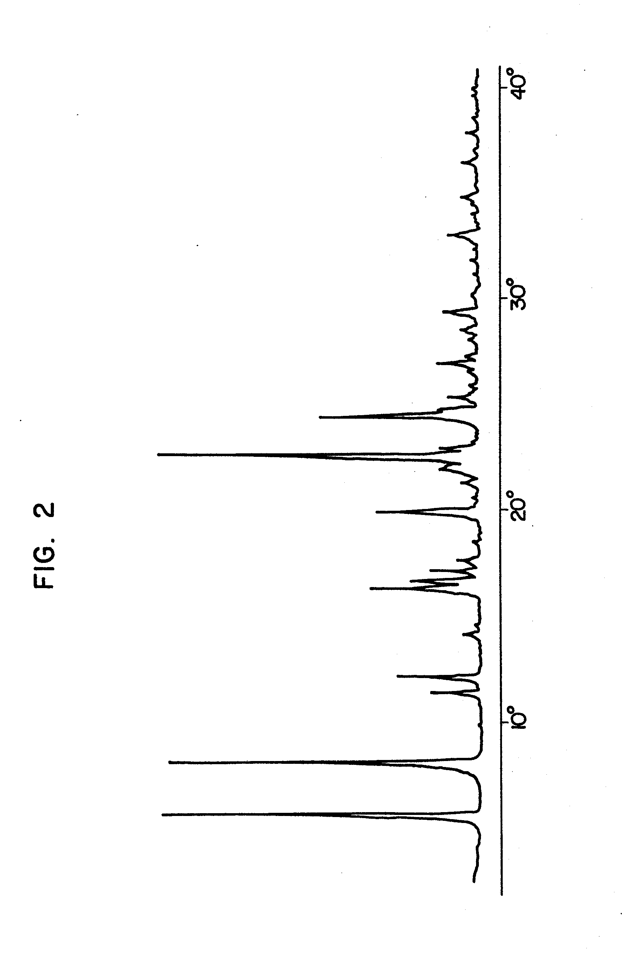

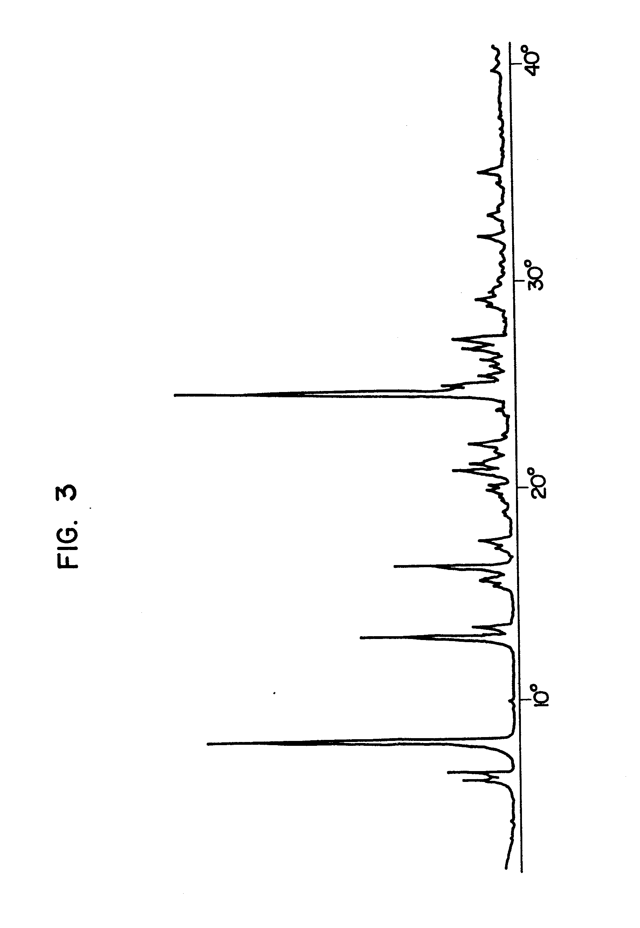

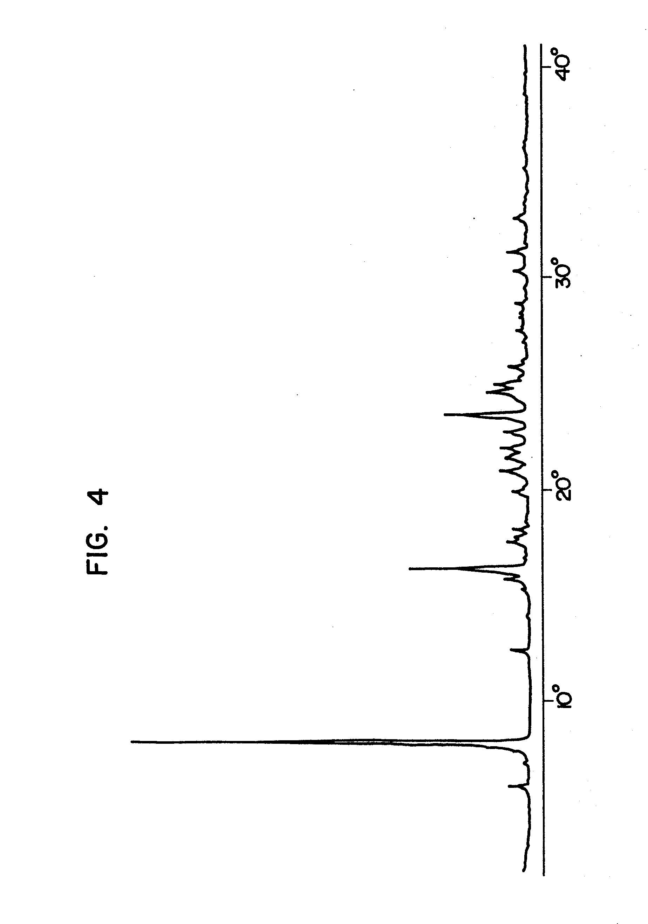

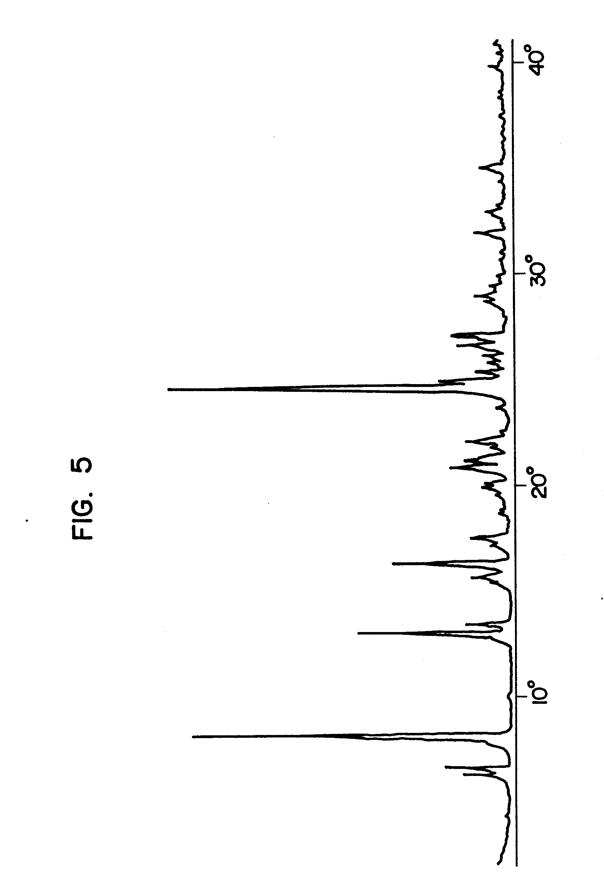

Of four these isomers, (+)(+) and (-)(-) are enantiomeric to each other, and the same applies also to (-)(+) and (+)(-). When a mixture of the same amounts of (+)(+) and (-)(-) is recrystallized, a racemic compound is produced, and similarly, the same amounts of (-)(+) and (+)(-) also produce the same racemic compound. From the powder X-ray diffraction patterns in Figs. 2 to 5 shown later and infrared absorption spectra, this compound turned out to be a complex salt comprising (+)-ICPA, (-)-ICPA, (+)-PTE and (-)-PTE, different compounds from the materials described above, in a molar ratio of 1 : 1 : 1 : 1 [hereinafter, said complex salt is abbreviated as (±)(±)]. From other combinations of two isomers than those described above, that is, from those of (+)(+) and (-)(+), (-)(-) and (+)(-), (+)(+) and (+)(-) and (-)(-) and (-)(+), the formation of complex salt is not found. As shown in Fig. 1, with respect to the solubility of said complex salt, (±)(±) has the smallest solubility in, for example, a 90% methanol solvent (containing 10% of water), and (-) (+) has the largest solubility in such solvent, which is much different from those of (±) (±) and (+) (+). This tendency is also the same with other solvents.

We find therefore that, provided that the optical purity of the (+)-PTE or (-)-PTE is sufficiently high to avoid significant formation of the abovementioned enantiomeric isomers of ICPA-PTE (and so prevent crystallization of the abovementioned racemic 1:1:1:1 complex compounds), then a (+)-ICPA-(+)-PTE or (-)-ICPA-(-)-PTE salt of high optical purity can be obtained. Thus, where the purity of the (+)-PTE or (-)-PTE is at least 95%, then the corresponding optically active salt can be obtained in high purity. There is thus no need for repeated purification of the optically active PTE before it is employed in an optical resolution method of the present invention.

Typically the optically active PTE may have an optical purity within the range 95-98.5%.

Next, the operating condition of the present invention will be illustrated with reference to a method to obtain (+)-ICPA using (+)-PTE.

First, ICPA is reacted with (+)-PTE to form a salt. As to the optical purity of (+)-PTE used here, the higher, the more preferred. Repetition of purification, which is not always advantageous in industry, is necessary to obtain a substantially pure (+)-PTE, however, and (+)-PTE having an optical purity of not less than 95%, preferably not less than 97%, can be used.

The amount of (+)-PTE is in a range of 0.5 to 1.0 mole, more preferably 0.6 to 0.8 mole, based on 1 mole of ICPA.

A temperature at which said reaction is carried out is optional, but for raising the optical purity of (+)-ICPA to a higher level, it is preferred to once heat to 40°C to 150°C and maintain the temperature on or after the reaction.

On said reaction, the use of inert solvent is preferred in carrying out the reaction smoothly, but it is not always necessary for the solvent to be the same as that used on crystallization and separation described later.

On said reaction, if the heating and temperature maintenance are once carried out, it is not always necessary for the salt to be in complete solution.

Next, the ICPA-PTE salt thus formed is crystallized in the solvent. In this case, it is preferred that the solution is cooled slowly.

As the solvent used on this crystallization, there may be given for example lower alcohols such as methyl alcohol, ethyl alcohol, n-propyl alcohol, isopropyl alcohol, n-butyl alcohol, isobutyl alcohol, sec-butyl alcohol, tert-butyl alcohol, etc., and lower aliphatic ketones such as acetone, methyl ethyl ketone, etc. These solvents may be used in a mixture with water. Further, these alcohols, ketones and their mixtures with water may be used in mixtures with aromatic hydrocarbons (e.g. benzene, toluene, xylene), aliphatic ones (e.g. hexane, heptane, octane), alicyclic ones (e.g. cyclohexane, methylcyclohexane) or halogenated ones (e.g. chloroform, carbon tetrachloride, chlorobenzene). The amount of the solvent is preferably 1 to 15 times by weight.

A temperature at which this crystallization is carried out is 0°C to 60°C, more preferably 10°C to 30°C.

Next, the crystallized salt crystal is separated from the mother liquor by means such as filtration, decantation and the like.

The crystal of salt thus obtained may be directly used as a material of the production of the (+)-ICPA ester, but it is preferred to decompose the salt into (+)-ICPA or its alkali salt by the usual method with an acid (e.g. hydrochloric acid; sulfuric acid) or an alkali (e.g. sodium hydroxide, potassium hydroxide), and then to convert the (+)-ICPA or alkali salt to the ester of (+)-ICPA.

The (+)-ICPA obtained by the method above, even by itself, has a sufficiently high optical purity, but if necessary, its optical purity may be heightened by recrystallization on the stage of the salt or after decomposition of the salt into (+)-ICPA.

When (-)-ICPA of high optical purity is required, this object can be attained, as a matter of course, by carrying out completely the same operation using (-)-PTE of not less than 95% in optical purity.

Next, the present invention will be illustrated in more detail with reference to the following examples.

The (+)/(-) ratio of ICPA and PTE in the examples was obtained as follows: After decomposing the ICPA-PTE salt as usual, said ratio of ICPA was measured by the method described in M. Horiba et al., Agric. Biol. Chem., <u>43</u>, 2311 (1979) and

that of PTE by the method described in M. Horiba et al., Agric. Biol. Chem., <u>44</u>, 2987 (1980).

Example 1

191.40 Grams of 90% methanol (containing 10% of water) was added to 63.80 g of ICPA, and ICPA was dissolved with stirring. Thereafter, 41.21 g of (+)-PTE (optical purity, 98.4%) was added, and the mixture was refluxed with heating. After 2 hours' refluxing, the reaction solution was cooled at a rate of about 1°C/5 minutes, and after the temperature reached 20°C, the solution was kept at the same temperature for 1 hour. The deposited crystal was collected by filtration, washed with a suitable amount of 90% methanol and dried to obtain a (+)-ICPA-(+)-PTE salt of the following composition.

At the same time, a comparative experiment was carried out in the same manner as above except that (+)-PTE of 91.6% in optical purity was used in place of the (+)-PTE of 98.4% in optical purity. The (+)-ICPA-(+)-PTE salt thus obtained was compared with that obtained above.

Result

| Optical purity of (+)-PTE used | | [Method of the present invention]<br>98.4% | [Comparative experiment]<br>91.6% |
|---|---|---|---|
| (+)-ICPA-<br>(+)-PTE<br>salt obtain-<br>ed | Yield (amount)<br>Yield (%)* | 47.32 g<br>74.4% | 49.86 g<br>78.2% |
| | ICPA (+)/(-)<br>(Optical purity) | 97.4/2.6<br>(94.8%) | 90.4/9.6<br>(80.8%) |
| | PTE (+)/(-)<br>(Optical purity) | 100.0/0.0<br>(100.0%) | 95.2/4.8<br>(90.4%) |

*Based on (+)-ICPA contained.

60.00 Grams of 90% methanol was added to 30.00 g of the (+)-ICPA-(+)-PTE salt obtained in the comparative experiment, and the mixture was refluxed with heating. After 2 hours' refluxing, the reaction solution was cooled to 20°C in 2 hours and kept at the same temperature for 1 hour. Thereafter, the crystal was collected by filtration, washed with a suitable amount of 90% methanol and dried to obtain 26.37 g of a (+)-ICPA-(+)-PTE salt.

Yield: 87.9% (based on the salt fed) ICPA (+)/(-) = 95.0/5.0 Optical purity of (+)-ICPA is therefore 90.0%.

PTE (+)/(-) = 96.8/3.2 Optical purity of (+)-PTE is therefore 93.6%.

As the above experiment shows, when (+)-PTE of low optical purity was used, a remarkable improvement in the optical purity of both (+)-ICPA and (+)-PTE is not observed even by recrystallization.

Example 2

21.27 Grams of ICPA was dissolved in 230.27 g of 90% methanol (containing 10% of water) with stirring, and after adding 21.13 g of (+)-PTE (optical purity, 100%) at 50°C, the mixture was refluxed with heating. After 2 hours' refluxing, the reaction solution was cooled at a rate of about 1°C/5 minutes, and one spatulaful of a (+)-ICPA-(+)-PTE salt was added as seed crystal at 60°C during cooling. After the temperature reached 20°C, the reaction solution was kept at the same temperature for 1 hour. The deposited crystal was collected by filtration, washed with a suitable amount of 90% methanol and dried to obtain 17.33 g of a (+)-ICPA-(+)-PTE salt.

Yield: 81.8% [based on (+)-ICPA contained] ICPA (+)/(-) = 97.7/2.3 Optical purity of (+)-ICPA is therefore 95.4%.

PTE (+)/(-) = 100.0/0.0

Example 3

A mixed solvent comprising 34.02 g of methanol, 8.51 g of water and 87.07 g of toluene was added to 63.80 g of ICPA, and the ICPA was dissolved with stirring. Thereafter, 41.21 g of (+)-PTE (optical purity, 95.4%) was added at 50°C, and the mixture was refluxed with heating. The subsequent procedure was carried out in the same manner as in Example 1 except that 80% methanol (containing 20% of water) was used for washing the crystal after filtration. Thus, 42.91 g of (+)-ICPA-(+)-PTE salt was obtained.

Yield: 67.4% [based on (+)-ICPA contained] ICPA (+)/(-) = 98.2/1.8 Optical purity of (+)-ICPA is therefore 96.4%.

PTE (+)/(-) = 100.0/0.0

Example 4

63.80 Grams of ICPA was dissolved in a mixed solvent comprising 114.84 g of ethanol, 12.76 g of water and 127.60 g of toluene, and after adding 41.21 g of (+)-PTE (optical purity, 97.2%), the mixture was refluxed with heating. The subsequent procedure was carried out in the same manner as in Example 1 except that 90% ethanol (containing 10% of water) was used for washing the crystal after filtration. Thus, 44.51 g of a (+)-ICPA-(+)-PTE salt was obtained.

Yield: 70.0% [based on (+)-ICPA contained] ICPA (+)/(-) = 97.2/2.8 Optical purity of (+)-ICPA is therefore 94.4%.

PTE (+)/(-) = 100.0/0.0

Brief Explanation of the Drawing:

Fig. 1 shows the solubility of a complex salt comprising (+)-ICPA, (-)-ICPA, (+)-PTE and (-)-PTE in a molar ratio of 1 : 1 : 1 : 1, a (+)-ICPA-(+)-PTE salt and a (-)-ICPA-(+)-PTE salt in a 90% methanol solvent (containing 10% of water). The curves ①, ② and ③ correspond to the above salts, respectively. In the figure, the ordinate indicates the solubility (wt.%), and the abscissa a temperature (°C).

Figs. 2 to 5 show the powder X-ray diffraction pattern of a mixture comprising the same amounts of a (+)-ICPA-(+)-PTE salt and a (-)-ICPA-(-)-PTE salt, a crystal obtained by recrystallization of said mixture, a mixture comprising the same amounts of a (-)-ICPA-(+)-PTE salt and a (+)-ICPA-(-)-PTE salt and a crystal obtained by recrystallization of said mixture. In the figure, the ordinate indicates a relative strength and the abscissa 2θ (θ, diffraction angle).

CLAIMS:

1.      A method for optical resolution of α-isopropyl-p-chlorophenylacetic acid (ICPA) which comprises reacting the α-isopropyl-p-chlorophenylacetic acid with (+)- or (-)-α-phenyl-β-(p-tolyl)ethylamine (PTE) of not less than 95% in optical purity to selectively crystallize the (+)-α-phenyl-β-(p-tolyl)ethylamine salt of (+)-α-isopropyl-p-chlorophenylacetic acid or the (-)-α-phenyl-β-(p-tolyl)ethylamine salt of (-)-α-isopropyl-p-chlorophenylacetic acid, and then collecting and decomposing the resulting salt to obtain (+)- or (-)-α-isopropyl-p-chlorophenylacetic acid.

2.      A method according to claim 1, wherein the (+)-PTE isomer is employed to yield (+)-ICPA.

3.      A method according to claim 1 or claim 2, wherein the optical purity of the (+)- or (-)-PTE is at least 97%.

4.      A method according to claim 1 or claim 2, wherein the optical purity of the (+)- or (-)-PTE is from 95-98.5%.

5.      A method according to any one of the preceding claims, wherein the solvent is a polar solvent or a mixture thereof with a hydrophobic solvent.

6.      A method of preparing an optically active isomer of ICPA, which method comprises reacting a racemic ICPA with (+)- or (-)-PTE having an optical purity of not less than 95%, allowing the resultant respective (+)-ICPA-(+)-PTE or (-)-ICPA-(-)-PTE salt to crystallize, and either directly trans-esterifying the resultant salt or decomposing the said salt into (+)-ICPA or (-)-ICPA respectively, or an alkali salt thereof and then esterifying the resultant said ICPA or alkali salt thereof.

7.        A method for optical resolution of racemic ICPA which comprises reacting the racemic ICPA with (+)- or (-)-PTE to yield (+)-ICPA-(+)-PTE or (-)-ICPA-(-)-PTE salt respectively, allowing the said salt to crystallize and then collecting and decomposing the said salt to obtain (+)- or (-)-ICPA, characterized in that the optical purity of the (+)- or (-)-PTE is not less than 95%.

FIG. I

FIG. 2

FIG. 3

FIG. 4

FIG. 5

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | JAPANESE PATENTS GAZETTE (DERWENT), vol. W27, 12th August 1975, abstract no. 45194 & JP - A - 50 025 544 (SUMITOMO CHEM IND K.K.) 18-03-1975 | 1 | C 07 C   57/58<br>C 07 C   51/487<br>C 07 B   19/00 |
| A | JAPANESE PATENTS GAZETTE (DERWENT), vol. X06, 17th March 1976, page 4, abstract no. 10328 & JP - A - 50 126 635 (SUMITOMO CHEMICAL K.K.) 04-10-1975 | 1 | |
| A | US-A-4 297 282   (OHASHI et al.)<br>* Column 4, lines 48-60 * | 1 | |

TECHNICAL FIELDS
SEARCHED (Int. Cl. ³)

C 07 C   57/00
C 07 C   51/00
C 07 B   19/00

The present search report has been drawn up for all claims

| Place of search<br>THE HAGUE | Date of completion of the search<br>23-01-1984 | Examiner<br>KLAG M.J. |
|---|---|---|

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503. 03.82